# EUROPEAN PATENT APPLICATION

(11) **EP 3 842 418 A1**
(43) Date of publication of application: **30.06.2021**
(21) Application number: 20216676.5
(22) Date of filing: 22.12.2020
(51) Int. Cl.: C07C 319/28, C07C 323/25

(54) **METHOD FOR THE PURIFICATION OF CYSTEAMINE**

(30) Priority: 23.12.2019 IT 201900025186
(71) Applicant: Farmabios S.p.A., 27027 Gropello Cairoli (Pavia) (IT)
(72) Inventor: Arosio, Roberto, 23862 Civate (LC) (IT); Zerilli, Francesco, 91020 Petrosino (TP) (IT); Gaboardi, Mauro, 28100 Novara (NO) (IT); Di Giacomo, Mario, 24069 Cenate Sotto (BG) (IT)
(74) Representative: Longoni, Alessandra

(57) **Abstract**

The present invention discloses a method for the purification of cysteamine or a salt thereof from polysulfuric impurities, in particular from cystamine, by treatment with dithiothreitol.

## Description

The present invention relates to a method for the purification of cysteamine or a salt thereof from polysulfuric impurities, in particular from cystamine.

Cysteamine is a known active ingredient used in therapy for treating cystinosis, a rare disease affecting cystine metabolism and appears in particular when the cystine transport system outside the lysosomes does not work, causing a buildup of cystine into the organism cells. This causes a malfunction of most organs. The first organs to be affected are kidneys and eyes, subsequently also thyroid, liver, muscles, pancreas and central nervous system become affected.

For the use in therapy, cysteamine is used in salified form, in particular as bitartrate or hydrochloride salt.

Several processes are known in the art for the synthesis of cysteamine.

For instance, EP 0 044 203 discloses a process for the synthesis of a cysteamine salt according to the scheme reported below. 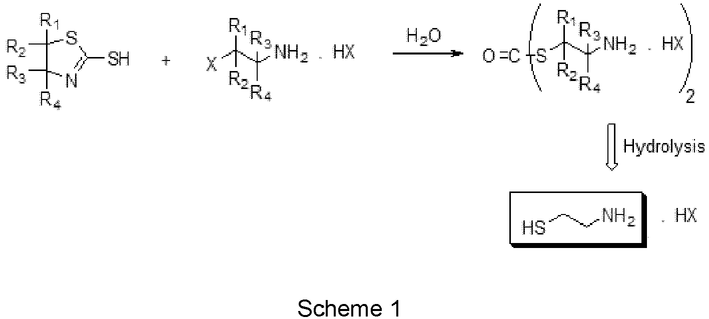 wherein R₁, R₂, R₃ ed R₄ can be the same or different from each other and each one represents a hydrogen atom, a low molecular weight alkyl group, also hydroxy substituted, or a phenyl group and X represents a halogen atom.

EP 0 054 409 discloses instead a process for the synthesis of cysteamine reported in the following scheme 2

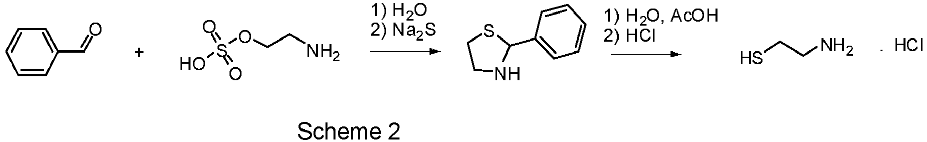

Furthermore, the Italian patent IT 1117224 describes a process for the synthesis of cysteamine reported here below (Scheme 3)

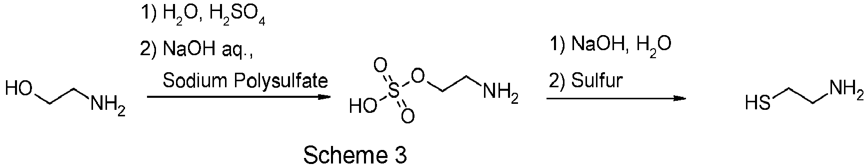

The processes for the preparation of cysteamine and salts thereof described above all present the issue of the purity of the final product since polysulfuric by-products, mainly cystamine are present in significant amounts. This involves the need of a complex final purification process of cysteamine.

In the Japanese patent JPS6087259, as a solution to the problem of the cystamine impurity, it is proposed a process for the preparation of cysteamine starting right from cystamine through reduction with hydrogen in presence of palladium. Such process however non only leads to an insufficient purification of cysteamine since a percentage of non-reacted cystamine remains but also introduces the necessity to purify the final product from palladium.

In the Japanese patent JPH05140087 is instead disclosed a method for the purification of cysteamine that provides for the distillation of cysteamine with water and/or an organic solvent, leaving polysulfuric impurities as non-volatile salts.

The use of distillation is however not advantageous, especially for industrial production on large scale.

We have now found a method for the purification of cysteamine or salts thereof that does not display the drawbacks of the known methods of purification and that allows to obtain cysteamine or salts thereof with a high purity without requiring procedures or reagents that make it laborious to use on an industrial level.

This method involves the purification of cysteamine or salts thereof by treatment of their aqueous solutions with dithiothreitol (DTT).

Thus, the method does not make use of organic solvents, laborious procedures or toxic reagents and allows to obtain cysteamine with a high degree of purity overcoming the drawbacks and disadvantages of the processes known in the art. Object of the present invention is therefore a method for the purification of cysteamine or a salt thereof from cystamine impurities comprising:
a) dissolving cysteamine or a salt thereof in water;
b) adding dithiothreitol to the resultant aqueous solution; and
c) isolating cysteamine or a salt thereof with a HPLC purity ≥ 98% from said solution.

A cysteamine salt according to the present invention is a pharmaceutically acceptable salt thereof, typically selected from hydrochloride, hydrobromide, tartrate, bitartrate, fumarate, succinate. Preferably, a cysteamine salt according to the present invention is the hydrochloride salt or bitartrate salt.

Cysteamine or a salt thereof to be purified according to the method object of the present invention has generally a purity of about 96-97%, with a content of cystamine of about 2%. Such degree of purity and such content of cystamine do not meet the standard acceptability requirements of products for pharmaceutical use. Cysteamine obtained after purification according to the method of the present invention has a degree of purity > 98%, for example > 99% or > 99.5% and a content of cystamine < 0.2%.

The degree of purity and the content of cystamine and of other potential polysulfuric impurities either in the product to be purified or in the purified product are determined by HPLC with column C18 in ion pair.

In step a) of the method object of the present invention, cysteamine or a salt thereof is dissolved in water.

The dissolution does not require particular conditions or precautions and is conveniently performed at room temperature. The amount of water is not a critical parameter even if from a practical point of view, it is suitable to avoid excessive dilutions or concentrations. Optimal is the dilution of 1 g of cysteamine or a salt thereof in a water volume comprised between 0.9 and 3 ml.

The amount of dithiothreitol added in step b) of the method object of the present invention varies from 0.02 and 0.4 equivalents with respect to the cysteamine dissolved in the solution.

In the present invention the term "dithiothreitol" means any isomeric form of the following compound:

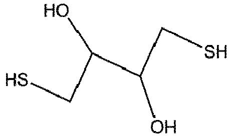

Also the addition of dithiothreitol does not require specific conditions and can be suitably performed at room temperature.

The resulting aqueous solution is then left under stirring, preferably at room temperature for several hours, generally between 6 and 24 hours, and then the purified cysteamine or a salt thereof are isolated from the solution according to conventional methods, preferably by crystallization.

In case cysteamine is obtained, this can be salified according to known methods maintaining the degree of purity thereof.

It is also possible, at the end of the purification method object of the present invention, to convert the obtained purified cysteamine salt into another salt maintaining the degree of purity thereof.

For instance, cysteamine hydrochloride having a degree of purity > 98% and a content of cystamine < 0.2% obtained according to the method of the present invention can be converted into the bitartrate salt according to known methods obtaining cysteamine bitartrate having a degree of purity > 98% and a content of cystamine < 0.2%.

In a preferred aspect of the present invention, the purification method is used to obtain cysteamine bitartrate having a degree of purity > 98% and a content of cystamine < 0.2%.

Said cysteamine bitartrate having a degree of purity > 98% and a content of cystamine < 0.2% can be obtained directly from cysteamine bitartrate having a content of cystamine < 0.2%.

Alternatively, said cysteamine bitartrate having a degree of purity > 98% and a content of cystamine < 0.2% can be obtained starting from cysteamine hydrochloride having a content of cystamine of about 2%, by converting purified cysteamine hydrochloride into cysteamine bitartrate.

The conversion of cysteamine hydrochloride into cysteamine bitartrate as disclosed in the art is preferably performed in presence of a base and tartaric acid, in a suitable solvent.

The purification method object of the present invention is thus a simple method, particularly convenient from an industrial applicability point of view since it does not require either the use of particular conditions or the use of toxic solvents or reagents. These features make it particularly suitable for the purification of cysteamine or a salt thereof deriving from any known process for the preparation of cysteamine.

The characterizing feature of the purification method object of the present invention is the use of dithiothreitol.

Even if dithiothreitol is a known reducing agent and thus, without being linked to any specific theory, it can be hypothesized that it acts as reducing agent for purifying cysteamine from the polysulfuric impurities, it is to be underlined that other well-known reducing agents usable in aqueous solutions, such as for instance sodium hydrosulfite, do not allow to obtain purified cysteamine.

This specific feature of dithiothreitol that cannot be observed in other known reducing agents make the method of the present invention particularly innovative.

The present invention will now be illustrated by means of some examples, that should not be seen as limiting the scope of the invention. All terms used in the present application, unless contrary indications, shall be understood in their common meaning as known in the art. Other more specific definitions for some terms, as used in this application, are underlined hereafter and apply for the whole description and set of claims, unless a different definition provides explicitly a broader definition.

### EXAMPLES

With reference to the following experimental part, the conditions in which HPLC analysis is performed in order to determine the degree of purity of cysteamine and the content of cystamine thereof are the following: column C18, mobile phase with ion pair, gradient.

### Example 1

### Purification of cysteamine hydrochloride from cystamine

In a 3-necked flask equipped with stirrer, cysteamine hydrochloride with about 2.5% of cystamine (4 g, 0.035 mol) was dissolved in demineralized water (12 ml) and dithiothreitol (1.09 g, 0.007 mol, 0.2 eq.) was added. The solution was left under stirring for 15-18 hours and controlled by HPLC. Content of residual cystamine 0.13%. Cysteamine hydrochloride was isolated by distilling water under vacuum and reintegrating with butanol (15 ml). After being left to crystallize under stirring, the precipitate was filtered, washed with butanol, dried at 50°C under vacuum until reaching a constant weight. 3.6 g of purified cysteamine hydrochloride were obtained with a content of cystamine 0.15%.

### Example 2

### Purification of cysteamine bitartrate from cystamine

In a 3-necked flask equipped with stirrer cysteamine bitartrate with about 2.5% of cystamine (5 g, 0.022 mol) was dissolved in demineralized water (12 ml) and dithiothreitol (0.88 g, 0.006 mol, 0.26 eq.) was added. The solution was left under stirring for 15-18 hours and controlled by HPLC. Content of residual cystamine <0.10%. Cysteamine hydrochloride was isolated by slow addition of isopropanol (70 ml). After precipitation, colling at 10°C, filtration and washing with isopropanol, the obtained solid was dried at 50°C under vacuum to give 4.5 g of purified cysteamine bitartrate with a content of cystamine 0.19%.

### Example 3

### Purification of cysteamine bitartrate from cystamine

In a 3-necked flask equipped with stirrer, cysteamine hydrochloride with about 2.5% of cystamine (13 g, 0.114 mol) was dissolved in demineralized water (30 ml) and dithiothreitol (1.76 g, 0.0114 mol, 0.1 eq.) was added. The solution was left under stirring for 15-18 hours and controlled by HPLC. Content of residual cystamine 0.20%. Cysteamine hydrochloride was then converted into cysteamine bitartrate by preparing a solution in isopropanol (175 ml) with tartaric acid (17.17 g, 0.114 mol, 1.0 eq.) and tributylamine (21.13 g, 0.114 mol, 1.0 eq.) and dropped it into the aqueous solution. After filtration of the obtained solid and washing with isopropanol, cysteamine bitartrate was obtained with a content of residual cystamine lower than 0.2%.

### Example 4 (Comparative)

### Attempt to purify cysteamine bitartrate from cystamine

In a 3-necked flask equipped with stirrer, cysteamine hydrochloride with about 2.5% of cystamine (3.0 g, 0.026 mol) was dissolved in demineralized water (10 ml) and sodium hydrosulfite (0.14 g, 0.00092 mol, 0.035 eq.) was added. The solution was left under stirring for 15-18 hours and controlled by HPLC. Content of residual cystamine 22%.

## Claims

1. A method for the purification of cysteamine or a salt thereof from cystamine impurities comprising:
a) dissolving cysteamine or a salt thereof in water;
b) adding dithiothreitol to the resultant aqueous solution; and
c) isolating cysteamine or a salt thereof with a HPLC purity ≥ 98% from said solution.

2. Method according to claim 1 wherein the cysteamine salt is the bitartrate salt or the hydrochloride salt.

3. Method according to claim 1 wherein dithiothreitol is added in an amount from 0.02 to 0.4 equivalents with respect to cysteamine.

4. Method according to claim 1 further comprising the salification of cysteamine obtained from step c).

5. Method according to claim 1 further comprising the conversion of the cysteamine salt obtained from step c) into another cysteamine salt.

6. Method according to claim 5 wherein cysteamine hydrochloride is converted into cysteamine bitartrate.

7. Method according to anyone of the previous claims wherein cysteamine or a salt thereof with a cystamine content < 0.2% is obtained.
